**Europäisches Patentamt**

**European Patent Office**

**Office européen des brevets**

(19)

(11) Publication number : **0 479 466 A2**

## (12) EUROPEAN PATENT APPLICATION

(21) Application number : **91308659.1**

(22) Date of filing : **24.09.91**

(51) Int. Cl.$^5$ : **G01N 33/00**

(30) Priority : **03.10.90 GB 9021472**

(43) Date of publication of application :
**08.04.92 Bulletin 92/15**

(84) Designated Contracting States :
**DE FR GB**

(71) Applicant : **British Nuclear Fuels PLC**
**Risley Warrington Cheshire, WA3 6AS (GB)**

(72) Inventor : **Ryden, David John**
**The Jordans, 5 Drayton Road**
**Sutton Courtenay, Oxon (GB)**
Inventor : **Waring, Stephen**
**6 Fettiplace**
**Wantage, Oxon OX12 7EN (GB)**

(74) Representative : **McCormack, Derek James**
**British Nuclear Fuels plc, Patents Section,**
**Legal Department**
**Risley, Warrington, Cheshire WA3 6AS (GB)**

(54) **Ruthenium monitor.**

(57)    An apparatus and a method are provided for monitoring the concentration of volatile radioactive ruthenium in a gas stream. The gas stream, after filtration to remove particulates, is passed through a packed column (18) in counter-current to a scrubbing liquid (26). The liquid is then collected, and gamma emissions from it are detected by a collimated detector (40). The apparatus is desirably of glass, and the liquid is desirably an aqueous sodium hydroxide solution.

EP 0 479 466 A2

This invention relates to an apparatus and to a method suitable for monitoring for the presence of radioactive ruthenium in a gas stream.

An off-gas stream from a nuclear fuel reprocessing plant or a radioactive material vitrification plant may contain ruthenium-106. Although the gas stream will have been treated to remove ruthenium it is desirable to be able to monitor the gas stream after such treatment to ensure correct operation of the treatment process. Ruthenium passes into the gas stream in three forms: as aerosols, as dry particulates, and as volatile species. The volatile for, normally considered to be $RuO_4$, is thermodynamically unstable and converts to $RuO_2$ (non-volatile fine particulate) during its passage down the off-gas treatment system. Volatile ruthenium is also deposited on off-gas pipework as $RuO_2$, and spalling of this deposit results in flakes of particulate material containing ruthenium.

The particulate and the volatile forms of ruthenium exhibit quite different decontamination factors in the off-gas treatment process. Effective monitoring of ruthenium therefore requires that both the particulate and volatile forms are monitored after treatment and before discharge of the gas. Particulate ruthenium can be filtered from the gas stream and monitored by a standard beta-in-air monitor. The present invention relates to the separate monitoring of the volatile ruthenium in a sample flow from which the particulate component is first removed.

According to the present invention there is provided an apparatus for monitoring for the presence of volatile radioactive ruthenium in a gas stream, the apparatus comprising a contacting chamber, means for causing at least part of the gas stream to flow through the contacting chamber, and means for causing a scrubbing liquid to flow through the contacting chamber and so contact the gas such that volatile ruthenium from the gas dissolves in the liquid, a collection chamber for collecting the liquid after its passage through the contacting chamber, and means for detecting ionizing radiation emitted from the liquid in the collection chamber.

Preferably the contacting chamber comprises a packed column through which the gas and the scrubbing liquid flow in counter-current. The scrubbing liquid is preferably an aqueous solution of sodium hydroxide, and is desirably recirculated through the contacting chamber and the collection chamber. Preferably substantially all the surfaces of the apparatus exposed to the liquid are of glass, preferably borosilicate glass, as this has been found to minimize the deposition of radioactive material on the surfaces.

The scrubbing liquid is preferably replaced at intervals, for example every day, as it may react with other gases in the gas stream, for example nitrogen oxide or carbon dioxide, and become less effective as an absorber of volatile ruthenium. It is also desirable to provide means to minimize evaporation from, or condensation to, the scrubbing liquid during operation, with consequential change in the volume of the scrubbing liquid. This may consist of means for maintaining the temperature of the liquid at the same temperature as the gas stream. Alternatively, there may be provided a top-up system to provide direct control of the liquid level.

The invention also provides a method for monitoring for the presence of volatile radioactive ruthenium in a gas stream, the method comprising contacting at least a portion of the gas stream with a scrubbing liquid such that volatile ruthenium dissolves in the liquid, collecting the liquid after it has contacted the gas, and monitoring the emission of ionizing radiation from the collected liquid.

Ruthenium-106 itself decays by a single beta-emission of maximum energy 39.4 keV, with a half-life of 368.2 days. Monitoring of the activity is more readily achieved by monitoring the daughter product rhodium-106, which has a range of beta transition energies from 1540-3541 keV and gamma emissions from 511.85 to 1562.2 keV. The most abundant gamma rays are 511.85 and 621.84 keV, and the half-life is 29.92 seconds. Hence it is preferably these gamma rays which are detected.

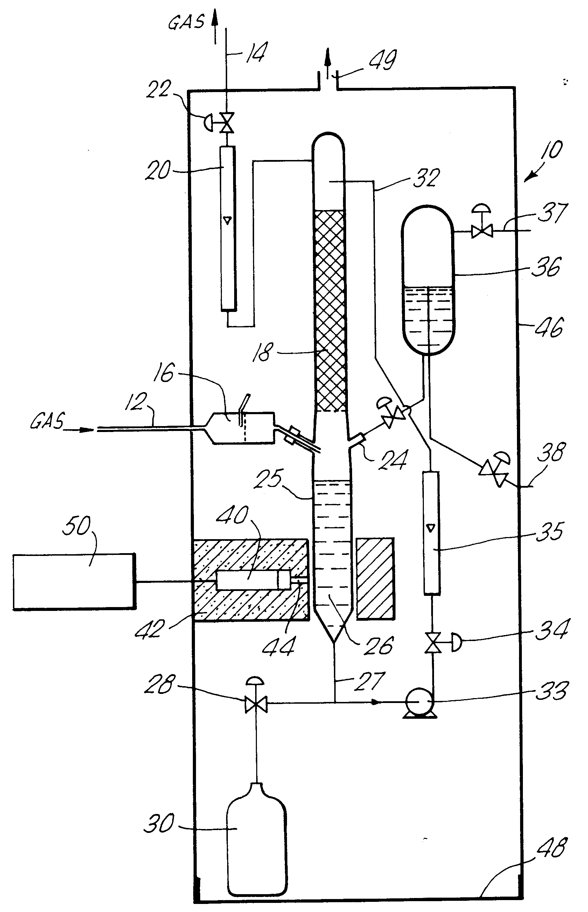

The invention will now be further described, by way of example only, and with reference to the accompanying drawing which shows a diagrammatic side elevation, partly in section, of an apparatus 10 for monitoring the concentration of volatile radioactive ruthenium in a gas. A steady flow of 37 litres/min of the gas to be monitored flows into the apparatus 10 through an inlet duct 12 and is extracted through an extract duct 14. Within the apparatus 10 the gas flows firstly through an in-line 40 mm diameter card-mounted particulate filter unit 16 (such a filter unit being available from Bird and Tole Ltd.), and then flows upwardly through a packed column 18, then through a flow meter 20 and a valve 22 to the extract duct 14.

The packed column 18 is a glass tube of internal diameter 80 mm, packed with 15 mm glass Raschig rings over 0.5 m of its length. Below the packed column 18 is the inlet for the gas and a valved inlet 24 for fresh liquid; the glass tube forms a three-litre collecting chamber 25 of internal diameter 100 mm below these inlets. The chamber 25 contains 3 litres of 5% (by weight) sodium hydroxide aqueous solution 26. An outlet duct 27 for the liquid 26 emerges from the base of the chamber 25 to communicate via a valve 28 to a spent liquid container 30; and the duct 27 connects to a recirculating duct 32 incorporating a seal-less, magnetically-coupled, PTFE-bodied pump 33, a valve 34 and a flowmeter 35, whereby the liquid 26 is supplied to the top of the packed column 18 at a nominal rate of 2.5 litre/min. Fresh solution is stored in a feed header tank 36 connected to the valved inlet 24, and provided with an inlet duct 37 and an overflow duct 38.

A sodium iodide gamma-ray detector 40, of diameter 76 mm and thickness 76 mm, is arranged 50 mm above the base of the chamber 25, surrounded by lead shielding 42 of thickness 50 mm including a collimator 44 defining a collimating hole so the detector 40 receives gamma rays from the contents of the chamber 25.

All the above components are installed in a cubicle 46 of dimensions 0.8 m wide, 0.6 m deep, and 2.0 m high, with transparent walls on all four vertical sides, incorporating doors in one wall. At the base of the cubicle 46 is a stainless steel drip tray 48 to retain any spilt liquids. In the top of the cubicle 46 is an air extract duct 49 whereby a steady flow of ambient air provides ventilation of the cubicle 46. The gamma ray detector 40 is connected to a suitable power supply and signal analysis and display unit 50, outside the cubicle 46.

In operation of the apparatus the scrubbing liquid 26 in the collecting chamber 25 is continuously recirculated through the packed column 18, flowing over the Raschig rings in countercurrent to the gas. Any volatile ruthenium ($RuO_4$) in the gas dissolves in the liquid 26. This process is very efficient, and typically the decontamination factor has been found to be well over 200. The presence of nitrogen oxides in the gas stream has not been found to affect the absorption of the ruthenium. Furthermore there has been found to be no measurable deposition of ruthenium onto any of the wetted glass surfaces of the apparatus 10; the ruthenium oxide forms non-volatile species such as ruthenate and perruthenate, so that it does not tend to re-enter the vapour phase on recirculating. The rate at which the concentration of ruthenium in the liquid 26 increases is clearly a measure of the concentration of volatile ruthenium in the gas stream. This is measured by measuring the gamma emissions from the short-lived daughter product rhodium-106, which is in equilibrium with the dissolved ruthenium-106 in the chamber 25.

To ensure correct operation of the apparatus 10, there may also be provided magnetically or optically-triggered alarms for each flow meter 20 and 35 to indicate if the flow rate is too high or too low. There may also be sensors to indicate if the level of the liquid 26 in the chamber 25 falls too low (due to evaporation or leakage) or rises too high (due to condensation), and these sensors may be linked to alarms, or to automatic means to supply or to withdraw liquid 26 from the chamber 25 and so maintain the level between set limits. A pressure monitor may also be provided to monitor the pressure drop across the filter unit 16, which can give an alarm if there is failure of the filter material.

Typically once a day the entire volume of scrubbing liquid 26 is changed, the spent liquid being drained into the container 30 and a fresh supply of 3 litres of sodium hydroxide solution being supplied from the header tank 36. The spent liquid is desirably analysed off-line, so as to provide retrospective confirmation of the measurements taken by the apparatus 10. Desirably the filter card in the filter unit 16 is replaced while the liquid is being changed.

In operation, gamma rays emitted by rhodium-106 are detected by the detector 40. The detection efficiency can be adjusted by means of exchangeable lead collimators 44 so that it suits the requirements for monitoring a particular gas stream: the detection efficiency must be sufficiently high to achieve the required sensitivity at the lowest activity levels, but not so high as to cause count saturation at the highest activity levels. The range of activities over which the monitoring apparatus 10 is sensitive can also be adjusted by substituting a detector with a smaller sodium iodide crystal for the detector 40. Because as mentioned earlier the most abundant gamma rays from the rhodium-106 are 511.85 and 621.84 keV, the signal analysis unit 50 incorporates a pulse height selector to define a signal window from about 450 to 650 keV. The count-rate within this signal window is indicative of the concentration of rhodium-106 (and hence that of ruthenium-106), while the count-rates above and below this window may be used to estimate the background in the signal window.

## Claims

1. An apparatus for monitoring for the presence of volatile radioactive ruthenium in a gas stream, the apparatus comprising a contacting chamber, means for causing at least part of the gas stream to flow through the contacting chamber, and means for causing a scrubbing liquid to flow through the contacting chamber and so contact the gas such that volatile ruthenium from the gas dissolves in the liquid, a collection chamber for collecting the liquid after its passage through the contacting chamber, and means for detecting ionizing radiation emitted from the liquid in the collection chamber.

2. An apparatus as claimed in Claim 1 wherein the detecting means detects gamma rays emitted from the liquid.

3. An apparatus as claimed in Claim 2 wherein the detecting means detects gamma rays over the range 450 to 650 keV.

4. An apparatus as claimed in any one of the preceding Claims wherein the contacting chamber comprises a packed column through which the gas and the scrubbing liquid are arranged to flow in counter-current.

5. An apparatus as claimed in any one of the preceding Claims also comprising means to recirculate the scrubbing liquid through the contacting chamber and the collection chamber.

6. An apparatus as claimed in any one of the preceding Claims wherein the scrubbing liquid comprises an aqueous solution of sodium hydroxide.

7. An apparatus as claimed in any one of the preceding Claims wherein substantially all the surfaces of the apparatus exposed to the liquid are of glass, preferably borosilicate glass.

8. An apparatus as claimed in any one of the preceding Claims including means to maintain the volume of the scrubbing liquid in the collection chamber within desired limits.

9. A method for monitoring for the presence of volatile radioactive ruthenium in a gas stream, the method comprising contacting at least a portion of the gas stream with a scrubbing liquid such that volatile ruthenium dissolves in the liquid, collecting the liquid after it has contacted the gas, and monitoring the emission of ionizing radiation from the collected liquid.

10. A method as claimed in Claim 9 wherein the scrubbing liquid comprises an aqueous solution of sodium hydroxide, the contacting is carried out in counter-current, and the collected scrubbing liquid is recirculated to contact the said portion of the gas stream.

11. An apparatus for monitoring for the presence of volatile radioactive ruthenium in a gas stream substantially as hereinbefore described with reference to, and as shown in, the accompanying drawing.

12. A method for monitoring for the presence of volatile radioactive ruthenium in a gas stream substantially as hereinbefore described with reference to, and as shown in, the accompanying drawing.